# EUROPEAN PATENT APPLICATION

(11) **EP 4 364 723 A1**
(43) Date of publication of application: **08.05.2024**
(21) Application number: 23207154.8
(22) Date of filing: 31.10.2023
(51) Int. Cl.: A61K 9/00, A61K 9/16

(54) **SLOW-RELEASE PHARMACEUTICAL, DIETARY AND/OR FOOD FORMULATION AND PROCESS FOR MAKING SAID FORMULATION**

(30) Priority: 07.11.2022 IT 202200022899
(71) Applicant: Vifra S.r.L., 03013 Ferentino (FR) (IT)
(72) Inventor: FORTUNA, Virgilio, Ferentino (FR) (IT); CAPPUCCI, Francesco, Ferentino (FR) (IT); MEZZINA, Cosmo, San Giuliano Milanese (MI) (IT); SACCOCCIO, Stefania, Patrica (FR) (IT)
(74) Representative: Lunati & Mazzoni S.r.L.

(57) **Abstract**

A slow-release pharmaceutical, dietary and/or food formulation in granular form is provided comprising encapsulated granules each one thereof comprising a core comprising mineral salts in an amount between 85% and 95% by weight of said encapsulated granule and including at least iron and zinc, and a coating comprising low-melting fats in an amount between 5% and 15% by weight of said encapsulated granule and including fatty acid derivatives.

## Description

The present invention relates to a slow-release pharmaceutical, dietary and/or food formulation, and related making process, of the type specified in the preamble of the first claim.

In particular, the present invention relates to a formulation in granular form containing encapsulated active principles so as to allow the controlled release inside human bodies, preferably by guaranteeing the correct absorption even inside the large intestine.

As it is known, the intestinal microflora is a complex system formed by a plurality of ecological niches housing a bacterial population formed by hundreds of different species.

Such microorganisms, for example, can be distinguished into anaerobic bacteria such as clostridia, bacteroids and bifidobacteria; aerobic bacteria such as enterobacteria, enterococci, lactobacilli and staphylococci; mycetes such as yeasts and fungi; and protozoa.

The several hundreds of species of microorganisms, furthermore, develop mainly in the large intestine.

The intestinal immune system and its flora, then, can be considered as a real organ, generally indicated as intestinal microbiota, whose equilibrium is important for the purposes of a good health.

The main function of the intestinal microbiota substantially is that of protecting the human body against pathogens by carrying out a plurality of different activities: participating in the absorption of the nutrients in the diet; metabolizing drugs and oncogenic substances; influencing absorption and distribution of the adipose tissue; contributing to the perception of pain in epithelium; and contributing to accumulation of adipose tissue in liver.

The nourishment of intestinal microbiota, then, has high importance for human health.

However, most formulations currently on the market are not capable of vehiculating the active principles as far as the large intestine.

In particular, the known formulations are at most capable of guaranteeing the release of active principles at the absorption sites of stomach or, in the best cases, inside duodenum, that is the initial portion of the small intestine.

In this situation the technical task underlying the present invention is to devise a slow-release pharmaceutical, dietary and/or food formulation, and related making process, capable of obviating substantially at least part of the mentioned drawbacks. Within said technical task an important object of the invention is to obtain a slow-release pharmaceutical, dietary and/or food formulation, and related making process, which is capable of guaranteeing the release of the active principles inside the large intestine.

Another important object of the invention is to implement a slow-release pharmaceutical, dietary and/or food formulation, and related making process, allowing to nourish the intestinal microbiota effectively.

In conclusion, an additional task of the invention is to implement a slow-release pharmaceutical, dietary and/or food formulation, and related making process, allowing to improve the state of health of the user.

The technical task and specified objects are achieved by a slow-release pharmaceutical, dietary and/or food formulation, and related making process, as claimed in enclosed claim 1.

Preferred technical solutions are highlighted in the depending claims.

In the present document, the measurements, values, shapes and geometrical references (such as perpendicularity and parallelism), when associated to words such as "about" or other similar terms such as "approximately" or "substantially", are to be meant as excluding measurements errors or inaccuracies due to production and/or manufacturing errors and, above all, excluding a slight deviation from the value, measurement, shape or geometrical reference thereto it is associated. For example, such terms, if associated to a value, preferably designate a deviation not higher than 10% of the value itself.

Moreover, when used, terms such as "first", "second", "higher", "lower", "main" and "secondary" do not identify necessarily an order, a relation priority or relative position, but they can be simply used to distinguish more clearly components different from each other.

The measurements and data reported in the present text are to be considered, unless otherwise indicated, as performed under International Standard Atmosphere ICAO (ISO 2533:1975).

The slow-release pharmaceutical, dietary and/or food formulation, the present invention relates to, is preferably in granular form.

Moreover, the formulation can be used to implement half-finished and/or finished products.

Substantially, under the term "half-finished product" one relates to the formulation arranged to be subsequently mixed with other components or re-processed with, for example known, processing methods.

Under the term "finished product", instead, one relates to the formulation ready for packaging.

Therefore, the finished product is the product defined with the formulation according to the invention ready to be used by any user, the half-finished product is defined by the formulation according to the invention ready to be subsequently processed and/or mixed with other components to implement the finished product.

The finished products made with the present formulation are preferably used for nourishing and feeding the intestinal microbiota.

The formulation according to the present invention is preferably a finished product. The formulation, which as already said is in granular form, preferably comprises encapsulated granules.

Then, since the granules are encapsulated, they include a core and a coating.

In particular, the core includes mineral salts. The core could also consist of mineral salts only. The mineral salts, moreover, preferably include at least iron and/or zinc. In turn, the mineral salts too can consist indeed of iron and/or zinc. Still more in detail, the iron could be present in form of ferrous gluconate 2-hydrate. The zinc, if present, could be in form of zinc gluconate.

From a constitutive point of view, the mineral salts can be comprised within the encapsulated granule in an amount between 70% and 98%, more preferably between 85% and 95% by weight of the encapsulated granule. Still more in detail, the mineral salts could be present in an amount between 89% and 93% by weight of the encapsulated granule.

The coating of the encapsulated granule, instead, advantageously comprises low-melting fats.

The latter, in detail, preferably include fatty acid derivatives. Still more in detail, the low-melting fats could consist of the fatty acid derivatives. Moreover, the fatty acid derivatives preferably include, or consist of, mono-diglycerides. The mono-diglycerides, in detail, could be present in form of 40-55% glycerol monostearate.

From a constitutive point of view, the low-melting fats can be comprised inside the encapsulated granule in an amount between 2% and 30%, more preferably between 5% and 15% by weight of the encapsulated granule. Still more in detail, the low-melting fats could be present in an amount between 7% and 11% by weight of the encapsulated granule.

In order to obtain the above-described formulation, the invention comprises a new process for making the formulation.

In particular, the process according to the invention comprises pre-heating a container from ambient temperature to a first pre-determined temperature.

The container, for example, is a container made of steel of bin type including a jacket for the collection of the components to be processed.

For example, in the pre-heating step, the container can be heated from the ambient temperature to a temperature equal to approximatively 50°C.

Moreover, the process includes a step of mixing the mineral salts with the low-melting fats inside the container so as to make a base mixture. Preferably, the mixing occurs by means of a rotating blade for a time necessary to make the mixture to reach a second pre-determined temperature.

Suitably, the second pre-determined temperature coincides at least with the melting point of the low-melting fats. For example, then, the second pre-determined temperature can be greater than or equal to 60° and less than 100°, in particular between 63°C and 73°C.

During mixing, the rotating blade, or impeller, can be actuated with speeds comprised between 50 rpm and 100 rpm, for example 80 rpm. The mixing step, moreover, can include the crushing of the components of the base mixture by means of a crusher, or chopper, acting at speeds comprised between 650 rpm and 750 rpm, for example 700 rpm.

The base mixture, preferably, includes the components of the formulation, that is mineral salts in an amount between 70% and 98%, more preferably between 85% and 95% by weight of the base mixture and low-melting fats in an amount between 2% and 30%, more preferably between 5% and 15% by weight of the base mixture. Still in more detail, the base mixture can suitably include mineral salts in an amount between 89% and 93% by weight of the base mixture and low-melting fats in an amount between 7% and 11% by weight of the base mixture.

The process, then, comprises a step of kneading the base mixture. The base mixture is preferably kneaded until homogenisation by making to decrease the container temperature so as to maintain the base mixture stably at a temperature close to the second pre-determined temperature.

Under the term "close" it is meant that the temperature remains within tolerances of ± 10°C with respect to the second pre-determined temperature.

Even during kneading, the base mixture is subjected to the action of the rotating blade and of the crusher. With the purpose of kneading suitably the base mixture, preferably, the rotating blade can be actuated with speeds between 120 rpm and 240 rpm, for example 180 rpm. Preferably, the rotating blade is of helical type. Moreover, preferably the rotating blade defines an overall diameter between 2 dm and 8 dm, more preferably equal to 5 dm. In each case, advantageously, in order to knead correctly mineral salts and low-melting fats it is preferable that, regardless of the sizes of the rotating blade, the latter rotates with a tangential speed between 1 m/s and 10 m/s, more preferably between 3 m/s and 6.5 m/s. The crusher can continue to crush the components of the mixture at speeds identical or similar to the mixing step.

The process then comprises a step of cooling until ambient temperature. In this step, the mixture in the container can be left to rest, or, preferably, it can be subjected to the action of the rotating blade at limited speeds. Preferably, the rotating blade, in this step, has a speed lower than 50 rpm, for example 20 rpm.

The process, then, includes a milling step suitable to allow the transformation of the base mixture in the encapsulated granules which are part of the formulation.

At the end of the milling step, the formulation is in the state of finished product or half-finished product.

In each case, the containers, the rotating blades, the crushers and possible heating and cooling apparatuses are devices known to the person skilled in the art and they have no particular arrangements other than those depending from the type of machinery in use.

For example, the process according to the invention, or a portion thereof, can be made with a HSMG Rotocube 120 machine marketed by IMA^{®}.

In short, the Rotocube 120 machinery is a machinery which allows, by using the arrangements described for the process according to the invention, to process powders and mixtures of powders with the purpose of obtaining granulates with better technological features of sliding, compressibility index and other features important for the similar substances.

The above-mentioned machinery allows various types of processing: wet granulation, melt granulation, pellet formation.

Such types of processing are implemented within a closed container, having controlled temperature, in which the powders are subjected to the mixing, wetting, granulation and low-temperature drying processes.

In particular, the drying process is implemented under vacuum conditions, with cooling and subsequent milling.

The process according to the invention substantially provides a melt granulation procedure.

The steps described previously for implementing the process for making the formulation are preferably implemented in the order in which they are described.

The slow-release pharmaceutical, dietary and/or food formulation according to the invention achieves important advantages.

In fact, the slow-release pharmaceutical, dietary and/or food formulation, and related making process, is capable of guaranteeing the release of the active principles inside the large intestine.

Then, the slow-release pharmaceutical, dietary and/or food formulation, and related making process, allows to nourish the intestinal microbiota effectively.

In conclusion, the slow-release pharmaceutical, dietary and/or food formulation, and related making process, allows to improve the state of health of the user.

Within such scope all details can be replaced by equivalent elements and the materials, shapes and sizes can be any.

## Claims

1. A slow-release pharmaceutical, dietary and/or food formulation in granular form comprising encapsulated granules
and **characterised by** that each of said encapsulated granules comprises
- a core comprising mineral salts in an amount of 85% to 95% by weight of said encapsulated granule and including at least iron and zinc,
- a coating comprising low-melting fats in an amount between 5% and 15% by weight of said encapsulated granule and including fatty acid derivatives.

2. Formulation according to claim 1, wherein said mineral salts are in an amount between 89% and 93% by weight of said encapsulated granule and said low melting fats are in an amount between 7% and 11% by weight of said encapsulated granule.

3. Formulation according to any one of the preceding claims, wherein said core comprises said mineral salts.

4. Formulation according to any one of the preceding claims, wherein said mineral salts comprise iron and zinc.

5. Formulation according to any one of the preceding claims, wherein said low melting fats comprise fatty acid derivatives.

6. Formulation according to any one of the preceding claims, wherein said fatty acid derivatives include or consist of mono-diglycerides.

7. Process for making a pharmaceutical, dietary and/or food formulation any one of claims 1-6, comprising:
- pre-heating a container from ambient temperature to a first pre-determined temperature;
- mixing said mineral salts and said low melting fat within said container in such a way as to define a base mixture, by means of a rotating paddle for a time necessary to make said base mixture reach a second pre-determined temperature coinciding with the melting point of said low melting fat;
- kneading said base mixture with said rotating paddle until homogenisation by decreasing temperature of said container so as to maintain said base mixture stably at a temperature close to said second pre-determined temperature;
- cooling said container to ambient temperature.

8. Process according to any preceding claim, comprising a step of milling said base mixture so as to make said encapsulated granules.

9. Process according to any one of claims 7-8, wherein said base mixture includes mineral salts in an amount between 85% and 95% by weight of said base mixture and low melting fats in an amount between 5% and 15% by weight of said base mixture.

10. Process according to any one of claims 7-9, wherein said first pre-determined temperature is between said ambient temperature and 50° and said second pre-determined temperature is greater than or equal to 60° and less than 100°.

11. Process according to any one of claims 7-10, wherein said rotating blade is helical and, during said kneading, rotates with a tangential speed between 3 m/s and 6.5 m/s.
